# EUROPEAN PATENT SPECIFICATION

(11) **EP 1 849 464 B1**
(45) Date of publication and mention of the grant of the patent: **08.08.2012**
(21) Application number: 07251814.5
(22) Date of filing: 30.04.2007
(51) Int. Cl.: A61K 9/70, A61L 15/28, A61L 15/44, A61L 15/22, A61K 33/38, A61L 15/18, A61L 15/46

(54) **Wound dressings**
Wundverbände
Pansements pour plaies

(30) Priority: 28.04.2006 GB 0608437
(43) Date of publication of application: 31.10.2007
(73) Proprietor: Advanced Medical Solutions Limited, Winsford, Cheshire CW7 3PD (GB)
(72) Inventor: Freeman, Richard, Christleton, Chester, Cheshire, CH3 7AH (GB); Bradford, Colin, Winsford, Cheshire, CW7 3PD (GB); Clarke, Roger, Winsford, Cheshire, CW7 3PD (GB)
(74) Representative: Atkinson, Peter Birch

(56) References cited:
- EP-A2- 0 255 248
- EP-A2- 0 272 149
- WO-A-02/36866
- WO-A-02/060501
- WO-A1-01/24839
- US-A1- 2002 073 891

## Description

The present invention relates to a method of producing alginate fibres for use in the production of wound dressings incorporating silver for delivery to a wound.

It is well known that silver has antimicrobial properties and is useful for preventing or inhibiting colonisation of wounds by bacteria that would have a deleterious effect on the healing of the wound. As such, silver has been incorporated both in metallic and "compound" form in various wound dressings so that the silver is delivered to the wound when the dressing is in contact therewith.

Thus, for example, WO-A-02062403 (Coloplast) discloses wound dressings having an absorbing element or constituent containing a complex of silver and a transition element of Group IV of the Periodic Table for providing silver to be delivered to a wound. The preferred complex silver sodium hydrogen zirconium phosphate, which is commercially available as AlphaSan ® (ex Milliken) with a silver content of about 10% by weight. Similarly WO-A-0236866 (SSL) discloses a wound dressing comprising alginate fibres incorporating AlphaSan ® once again for delivering silver to a wound.

However such complexes are expensive and this problem is compounded by the fact that they have relatively low silver contents. Therefore a relatively large amount of the (expensive) complex may be required to achieve a desired silver level in the wound dressing.

WO 01/24839A discloses products containing a stabilised form of silver. The products, which may be used as wound dressings, comprise a polymer network and a non-gellable polysaccharide containing a silver compound such as silver chloride, silver phosphate, silver iodide, and silver bromide.

EP 0 255 248 A discloses an antimicrobial wound dressing prepared by vapour or sputter coating certain silver salts (preferably silver chloride and silver sulphate) onto a wound dressing substrate.

US 2002/0073891 discloses the preparation of materials containing stabilised silver for use, for example, as wound dressings. The material comprises a polymer incorporating the silver and is prepared by treatment of the polymer with a solution comprised of an organic solvent and a source of silver. The silver source may, be a silver salt, for example silver nitrate, silver chloride, silver bromide, and silver carbonate, amongst others.

EP 0 272 149 A discloses a medical dressing comprising, in addition to a water-tight film, a sealing material which is comprised of a continuous phase comprising a tackifier and a discontinuous phase (dispersed in the continuous phase) which comprises a water-soluble or water-swellable hydrocolloid, starch, cellulose derivative or hydrophilic polymer. The dressing may incorporate an antiseptic agent, for example sulphadiazine silver or silver chloride.

WO02/060501 A discloses a hydrogel wound dressing which may optionally incorporate an antimicrobial or bacteriastatic agent, for example a silver salt such as silver acetate, silver benzoate, silver carbonate, silver iodide, and silver oxide (amongst others).

It is therefore an objection of the present invention to obviate or mitigate the above mentioned disadvantages.

According to the present invention there is provided a method producing alginate fibres comprising the steps of:
(i) preparing an aqueous spinning dope containing a dissolved alginate polymer and a dispersion of a particulate, water-insoluble, inorganic silver salt containing at least 50% by weight of silver; and
(ii) spinning said dope into a coagulating bath containing multi-valent cations for effecting cross-linking of the alginate chains.

By "water-insoluble" we mean that the silver salt has a solubility constant at 25°C of 1.4 x 10⁻⁵ or less. A list of solubility constants of silver salts may be found at http://www.stolaf.edu/people/hansonr/jscal/jsca/c2.htm.

Fibres manufactured in accordance with the method of the invention are useful as the wound contacting material of wound dressings.

The use of insoluble, inorganic salts which themselves contain at least 50% by weight of silver has a number of advantages for providing delivery of silver from a dressing to a wound. In particular, the high silver content (i.e. greater than 50% by weight) in the silver salt means that much lower amounts of the salt are required to achieve a particular silver level than is the case for complexes such as AlphaSan ®. This itself provides a twofold advantage. Firstly, the salts are cheaper than the complexes so that for a given silver level in a dressing the cost of the silver-containing component is much less in the case of dressings in accordance with the invention than those containing complexes such as AlphaSan ®. Secondly the relatively lower amount of salts that are employed in the invention has particular advantage in the case of wound dressings for which the wound contact material is of a "relatively delicate" structure. Thus, for example, such a material may be comprised of fibres having a diameter of 20 microns (see also below), in which case it is a considerable advantage to have as low a content of the particulate silver-delivery component as possible because otherwise the strength characteristics of the fibres may be compromised so they are subject to breakage (particularly after sterilisation).

Additionally materials incorporating the silver salts may be subjected to aqueous washing procedures without any significant loss of silver content. Nevertheless when the material is in contact with wound fluids (e.g. exudates) ionexchange occurs (e.g. with sodium ions) whereby silver ions are delivered to the wound. This enables the silver ions to be delivered at a much slower, more controlled rate than in the case of water-soluble salts. This slower, controlled release of silver gives the wound dressing a 'reservoir' of silver which extends the duration of the therapeutic effect. The dressing may therefore be left in place for prolonged periods and will remain an effective antimicrobial dressing. This is particularly advantageous in the case of burn patients.

Further advantages are that the silver salts are generally thermally stable and can be used in manufacturing processes requiring the use of elevated temperature.

A further potential advantage of the use of silver salts is one of possible electrical conductance to allow use of electrical stimulation to improve wound healing.

Generally the silver salt will have a particle size less than 5 microns, more usually less than 2 microns, although optimal particle size may be dependent on factors such as the nature of the wound contacting material of the dressing. Thus, for example, if the wound contacting material is comprised of relatively delicate fibres having a diameter of, say, 20 microns.then the particle size of the silver salt may well be somewhat lower and for the case where the wound contacting region is of a more robust structure. In the case of fibres, the particle size of the silver salt should be less than 20% of the fibre diameter.

Silver salts used in the invention contain at least 50% by weight silver. This percentage is calculated on the basis of the chemical formula of the salt but excluding any water of crystallisation. Preferably the silver salt contains at least 60% more preferably at least 70% and ideally at least 76% by weight of silver. The preferred silver compound is silver carbonate which has a silver content of about 78% by weight (calculated on the basis of the chemical formula being Ag₂CO₃). Silver carbonate is preferred because it incorporates a biocompatible cation (i.e. the carbonate ion), it is stable to all typical manufacturing processes used for wound dressings and also stable to sterilisation by steam, ethylene oxide or gamma-irradiation. Other silver salts that may be employed include silver (I) oxide, silver chloride, silver sulphide and silver phosphate which have respective silver contents of about 93%, 75%, 87% and 77% respectively.

The amount of the silver salt in the wound contacting material of the dressing will of course determine the actual silver content of the wound contacting material and will depend on factors such as the nature of the wound to be treated and the type of material from which the wound contacting region of the dressing is fabricated. In principle however, the amount of the silver salt should not be too low otherwise the antimicrobial performance of the dressing may not be sufficient. Conversely the amount of the silver salt should not be so high that there may be a significant detrimental impact on wound healing. Typically the amount of the silver salt will be such as to provide a silver content for the wound contacting material of 0.1-5% by weight. More typically, the amount of silver in the wound contacting material of the dressing will be in the range 0.2-3.0% by weight.

In addition to the silver salt, the wound contacting material may contain one or more other agents (e.g. salts, soluble or otherwise) to assist in the healing of wounds. Thus, for example, this agent may be zinc or selenium, e.g. provided by their oxides. Generally the amount of zinc and selenium in the material will each be in the range 0.1%-5.0% by weight, more typically 0.5 - 2.0%, e.g. about 1%.

The alginate may fibres may havea diameter of 15-20 microns (more preferably about 20 microns) although we do not preclude the use of fibres having a diameter outside this range.

The alginate used for production of the fibres may, for example have a G-content of 35-70% by weight and correspondingly an M-content of 65-30% by weight. Typically the alginate will be such that a 1% solution in water will have a viscosity of 30-300 cP, more preferably 40-100 cP.

The alginate fibres may be produced in known manner by spinning a dope comprised of sodium alginate dissolved in water into a coagulating bath incorporating a multivalent cation for effecting cross-linking of the alginate chains. The multivalent (cross-linking) cation will generally be calcium, and usually provided in the form of dissolved calcium chloride. Other multivalent cations have been used including barium. Generally the dope will have a total solids, dissolved and dispersed content, of less than 10% by weight preferably 5%-7%, e.g. about 6%.

The silver salt to be incorporated in the alginate fibres may be included in the coagulating bath but more preferably is included in the spinning dope.

Given that the alginate fibres will (as indicated above) have a preferred diameter in the range 15-20 microns then the particles of the silver salt and any other insoluble salt should have a size in the range 0.5 to 5 microns and more preferably less than 2 microns. These size limitations are to prevent breakage of the fibres due to incorporation therein of "large" particles,

If these fibres are to include another active material (e.g. a source of zinc or copper or selenium) then the source material may be included in the coagulating bath but, once again, is more preferably included in the spinning dope.

The alginate fibres may, with advantage, may incorporate at least one other water soluble polysaccharide (usually having negative charges along the chain) for increasing the absorbency of the resultant fibres. Such fibres may be produced by incorporating the alginate and the other water soluble polysaccharide in the dope for a co-spinning procedure in accordance with the disclosure of WO-A-9610106 (Innovative Technologies Limited). Such fibres may comprise (based on the dry weight of the fibre) 70-99% by weight of the alginate, and a total of 1-30% (e.g. 5-20%) by weight of at least one further polysaccharide, the silver salt and optional other components (e.g. zinc salts). The fibres may, for example, comprise 70-95% (e.g. 70-90%) by weight alginate, 3-20% (e.g. 5-20%) by weight of the at least One further polysaccharide and a balance of the silver salt and optional other components (e.g. zinc salts). Particularly suitable polysaccharides for use in this embodiment of the invention include Carboxy Methyl Cellulose (CMC), hydroxyproplymethylcellulose (HPMC), other cellulose derived absorbent materials, pectin, etc.

Fibres comprising an alginate and at least one further polysaccharide may be produced by the steps of:
(a) preparing a spinning dope containing less than 10% by weight of a solids mixture which comprises 70-99% by weight of alginate, 1-30% by weight of the at least one further polysaccharide and the silver salt; and
(b) spinning the dope into a coagulating bath incorporating a multivalent cation (preferably calcium) for effecting cross-linking of the alginate chains.

The solids mixture may for example comprise 70-95% (e.g. 70-90%) by weight alginate and 3-20% (e.g. 5-20%) by weight of at least one further polysaccharide.

Wound dressings prepared from fibres produced in accordance with the invention may take a number of forms. Thus, for example, the wound contacting material comprised of the alginate fibres (incorporating the silver salts) may be the sole component of the dressing. Such dressings may, for example be in the form of a flat dressings, ribbon or as a fibrous carded sliver. Alternatively the dressing may comprise the wound contacting material and a backing layer.

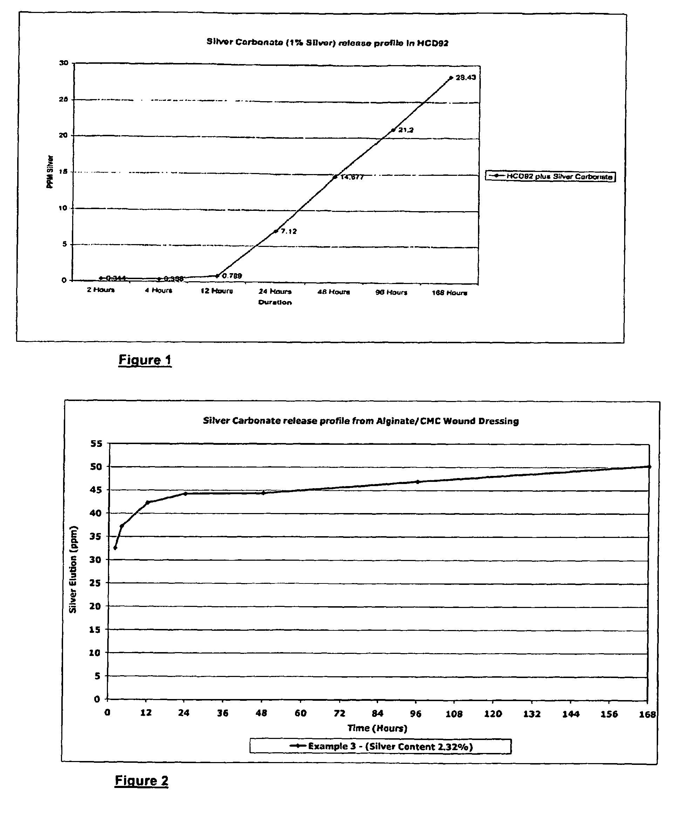

The invention will be illustrated by the following non-limiting Examples and Figs 1-4 of the accompany drawings which illustrates the results of the Examples.

### Example 1 - Silver Hydrocolloid (Reference Example)

This Example describes the production testing of a hydrocolloid wound dressing material in accordance with the invention.

### Preparation

A hydrocolloid wound dressing was prepared from the following components:

| **Component** | **Amount (g)** |
|---|---|
| SBS Block Copolymer | 125 |
| Pectin | 92.5 |
| Carboxy Methyl Cellulose | 150 |
| Butylated Hydroxytoluene | 2.5 |
| Purified Powdered Cellulose | 20 |
| Mineral Oil | 10 |
| Polyisobutylene | 100 |
| Silver Carbonate | 7 |

The above components were added to a 1 Litre Winkworth Z-blade mixer which was then started and heated to 70° C. After 15 minutes, the temperature of the mix was checked and confirmed to be 70° C. A sample of the mix was also taken at 15 minutes and pressed to a flat sheet having a thickness of about 0.4 mm. A visual inspection confirmed the uniformity of the flat sheet. After a total of 30 minutes mixing, temperature of the mixture was again confirmed to be 70° C and the mix was found to be uniform.

Mixing was terminated after 30 minutes and the resulting dope was compressed to form a flat sheet having a nominal thickness of 0.4 mm.

### Testing

(a) Silver Content: A 2.5x2.5m sample of the flat sheet was digested in mineral acids until it was completely dissolved. The silver content of the liquid was measured using Atomic Absorption Spectroscopy.
(b) Silver Elution: 7 Vials were prepared numbered 1 to 7 each containing a 2.5x2.5cm sample of the flat sheet in 10ml of simulated wound fluid (Na⁺ 140mM, Cl⁻ 109mM, K⁺ 4mM, Ca²⁺ 2.5mM, HCO₃⁻ 40mM, and Bovine Albumin 3.3g in 100ml). After 2 hours, a sample of the supernatant liquid from vial 1 was taken and the silver content measured using Atomic Absorption Spectroscopy. The vial and sample were discarded. The procedure was repeated at the following times, 4 hours, 12 hours. 1 day, 2 days, 4 days and 7 days. The results are shown in Figure 1.

### Results

The silver content of the film (as measured above) was found to be 1.05% by weight. This compares with a value of 1.38% based on the formulation shown in the above table. The result as determined by Atomic Absorption Spectroscopy is considered to be within experimental error.

It can be seen from Figure 1 that significant silver release began once the hydrocolloid matrix had become fully saturated (around 24 hours) and that silver release totalled 28 ppm after 7 days.

### Example 2 - Silver Alginate/CMC Fibres

The Example describes the production of fibres comprised of a mixture of sodium/calcium alginate and carboxymethyl cellulose (CMC).

### Preparation

An aqueous spinning dope was prepared containing 6% by weight of the following formulation:

| **Component** | **% By Weight** |
|---|---|
| Sodium Alginate | 85% |
| CMC | 13% |
| Silver Carbonate | 2% |

The alginate used was a High M (Mannuronic acid, 60% M) material which was selected because it is highly absorbent and forms a soft gel with wound exudates. The CMC improves the absorbency and speeds fluid uptake to allow an increased rate of gelling action.

The dope was prepared by initially mixing the silver carbonate with water until the silver compound was fully dispersed. The CMC and sodium alginate were then mixed with the water and silver carbonate until uniform. The dope was allowed to stand to allow air bubbles to escape.

The dope was filtered to remove large particles (filter size nominally 30microns) and then pumped through a spinnerette having 40,000 holes into a coagulant bath containing 2% w/v calcium chloride dihydrate.

The resulting coagulated fibres (the "tow") were passed through hot water (> 90°C) and stretched to orientate the alginate molecules.

The tow was washed with water to remove residual calcium chloride and sodium chloride formed from the ion exchange process. Subsequently the tow was passed through a series of acetone and water solutions to remove about 50% of the water from the tow.

In the next step, the tow was dried in a hot air oven to achieve a moisture content of about 20% by weight.

Polyethylene glycol 400 was then applied to the fibres at a level of 0.5-2% by weight to provide a spin finish.

The tow was then crimped and finally cut into fibres having a length of 50 mm.

The fibres could be made into a felt (for use as a wound dressing) using standard procedures. Allowing for the moisture content of the fibres, the dressing contained about 1.3% by weight of silver. The wound dressings obtained were highly absorbent and capable of delivering silver to a wound.

### Example 3 - Silver Alginate/CMC Fibres

The procedure of Example 2 was followed but using an aqueous spinning dope containing 6% by weight of the following formulation:

| **Component** | **% By Weight** |
|---|---|
| Sodium Alginate | 92% |
| CMC | 4.25% |
| Silver Carbonate | 3.75% |

The resultant wound dressings were highly absorbent.

The silver content as measured by the procedure described in Example 1 was found to be 2.32%.

Silver elution was measured as described in Example 1 and the results are shown in Figure 2.

The ability of the dressings to control MRSA (Methicillin Resistant Staphylococcus Aureus) was evaluated using a 21 Day Log Reduction method as detailed below.

Wound dressing pieces having a size of approximately 1.5 cm x 1.5 cm were added to a flask containing 20ml of simulated wound fluid (SWF) having the formulation described in Example 1.

0.2ml of a suspension of the MRSA at a nominal level of 1.0 x 10⁸ cfu/ml was added, giving a nominal level of 1.0 x 10⁶ cfu/ml in the SWF. This level equates to an infected wound.

Negative and positive controls were prepared in the same way. The negative control (no silver) was SeaSorb Soft™, a product comprised of 85% alginate and 15% CMC. The positive control was Aquacel Aq^{™}, a sodium carboxymethylcellulose primary wound dressing containing silver.

The micro-organism level was measured for all flasks by removing 0.5ml of the SWF at specified time points to perform a standard dilution series plate count The 0.5ml was replaced with SWF to maintain the volume. Sampling was effected at the following time points:

0, 1, 2, 4 & 6 hours on day 0, days 1, 2, 3, 4, & 7.

After the day 7 count, the dressings are challenged by adding 0.2ml of the 1.0 x 10⁸ suspension. Counts were then performed at:

0, 1, 2,4 & 6 hours on day 7, days 8, 9, 10, 11, & 14.

After the day 14 count, the dressings are challenged by adding 0.2ml of the 1.0 x 10⁸ suspension. Counts were then performed at:

0, 1, 2, 4 & 6 hours on day 14, days 15, 16, 17, 18, & 21.

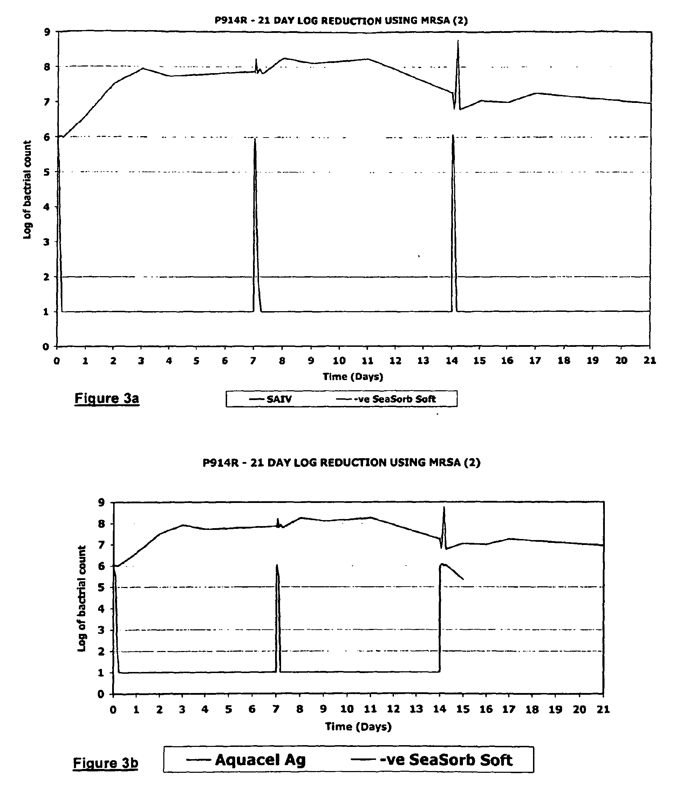

The results are shown in Figs 3a and 3b. Fig 3a shows the results for the negative control (SeaSorb Soft^{™} - upper trace) with those for the product of the inventions. Fig 3b shows the results for the negative control (SeaSorb Soft^{™} - upper trace) with those for the positive control (Aquacel Ag^{™}).

The results clearly demonstrate the effectiveness of the dressing in accordance with the invention in controlling MRSA in the SWF. More particularly (as shown in Fig 3a) the MRSA count peaked at 10⁶ after each inoculation of MRSA, (i.e. on days 0, 7 and 14) but then very rapidly dropped to a substantially constant value of 10. These results are to be contrasted with those obtained for the negative control, for which concentration of MRSA in the SFW was always above 10⁶.

The results for the product of the invention are to be further contrasted with those for the positive control (Aquacel Ag - Fig 3b). As shown in Fig 3b, the positive control was able to reduce the MRSA count very rapidly to a value of 10 after the MRSA inoculation on days 0 and 7. However after the inoculation on day 14 the MRSA count reduced only slowly so the test was terminated.

Thus the produce of the invention continued to kill MRSA to 21 days whereas the Aquacel Ag did not. In fact, Fig 3b demonstrates that Aquacel Ag did not produce significant kill from day 14 onwards. The benefit of the product of the invention is that the dressing can be left in place for prolonged periods aud will rental as effective antimicrobial dressing. This is particularly advantageous for treatment of burns.

### Example 4 - Silver & Zinc Alginate Fibres

This Example describes the production of fibres similar to those produced in Example 2 but additionally incorporating the zinc oxide as a source of zinc to increase the rate of wound healing.

### Preparation

An aqueous spinning dope was prepared containing 6% by weight of the following components:

| **Component** | **% By Weight** |
|---|---|
| Sodium Alginate | 85% |
| CMC | 11.5% |
| Silver Carbonate | 2.0% |
| Zinc Oxide | 1.5% |

The zinc oxide had a particle size of less than 1 µm to prevent weakening of the fibres.

The fibres were prepared using the procedure described in Example 2, wherein the zinc oxide was mixed into the water and silver carbonate before the CMC and alginate was added.

The resulting fibres were highly absorbent and (allowing for the moisture content) contained 1.3% silver and 1.0% zinc as measured in accordance with the procedure of Example 1.

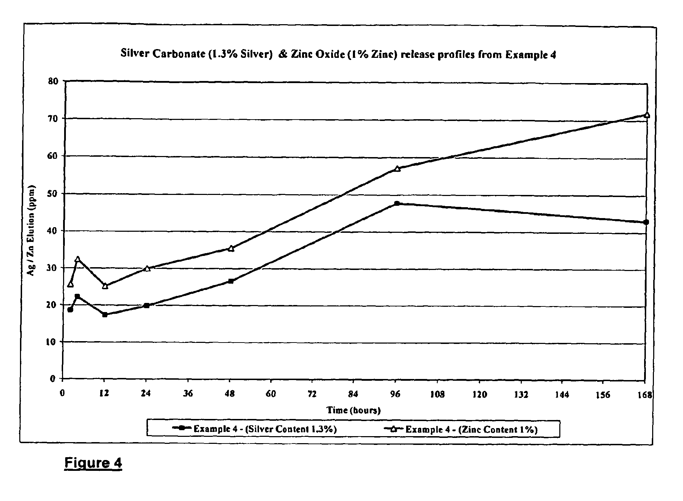

Silver and zinc elution were measured as described in Example 1. The results are shown in Figure 4.

## Claims

1. A method producing alginate fibres comprising the steps of:
(i) preparing an aqueous spinning dope containing a dissolved alginate polymer and a dispersion of a particulate, water-insoluble, inorganic silver salt containing at least 50% by weight of silver; and
(ii) spinning said dope into a coagulating bath containing multi-valent cations for effecting cross-linking of the alginate chains.

2. A method as claimed in claim 1 wherein the silver salt has a silver content of at least 76% by weight of silver.

3. A method as claimed in claim 2 wherein the silver salt is silver carbonate.

4. A method as claimed in any one of claims 1 to 3 wherein the alginate fibres contain 0.1-5% by weight of silver.

5. A method as claimed in claim 4 wherein the alginate fibres contain 0.2-3.0% by weight of silver.

6. A method as claimed in any one of claims 1 to 5 wherein the alginate fibres incorporate a salt or salts, additional to said silver salt.

7. A method as claimed in claim 6 wherein said additional salt is a zinc, copper or selenium salt.

8. A method as claimed in any one of claims 1 to 7 wherein the fibres comprise alginate and at least one other polysaccharide.

9. A method as claimed in claim 8 wherein the alginate fibres comprise 70-99% by weight of alginate and 1-30% by weight of said other polysaccharide.

10. A method as claimed in claim 9 wherein said other polysaccharide is Carboxy Methyl Cellulose.

11. A method as claimed in any one of claims 1 to 10 wherein said fibres have a diameter of 15-20 microns.

12. A method as claimed in claim 11 wherein the silver salt has a particle size of less than 5 microns.

13. A method as claimed in claim 12 wherein said silver salt has a particle size of less than 2 microns.

14. A method as claimed in claim 1 wherein the dope incorporates at least one further polysaccharide additional to the alginate.

15. A method as claimed in claim 14 comprising the steps of:
(a) preparing a spinning dope containing less than 10% by weight of a solids mixture which comprises 70-99% by weight of alginate, 1-30% by weight of the at least one further polysaccharide and the silver salt; and
(b) spinning the dope into a coagulating bath incorporating a multivalent cation (preferably calcium) for effecting cross-linking of the alginate chains.

16. A method as claimed in claim 14 or 15 wherein said further polysaccharide is Carboxy Methyl Cellulose.

17. A method as claimed in claim 1 wherein the dope is prepared by initially dispersing the silver salt and any other salt in water then dissolving the alginate and any other polysaccharide.

18. A method as claimed in claim 1 wherein the dope is prepared by initially dispersing the silver salt and a zinc salt and/or selenium salt and/or copper salt in water then dissolving the alginate and any other polysaccharide.

## Patentansprüche

1. Verfahren zum Herstellen von Alginatfasern, das die folgenden Schritte umfasst:
(i) das Bereiten einer wässrigen Spinnflussigkeit, die ein gelöstes Alginatpolymer und eine Dispersion eines teilchenförmigen, wasserunlöslichen, anorganischen Silbersalzes, das wenigstens 50 Gewichts-% an Silber enthält, enthält, und
(ii) das Spinnen der Spinnlösung in ein Erstarrungsbad, das mehrwertige Kationen enthält, um eine Vernetzung der Alginatketten zu bewirken.

2. Verfahren nach Anspruch 1, wobei das Silbersalz einen Silbergehalt von wenigstens 76 Gewichts-% an Silber hat.

3. Verfahren nach Anspruch 2, wobei das Silbersalz Silbercarbonat ist.

4. Verfahren nach einem der Ansprüche 1 bis 3, wobei die Alginatfasern 0,1 bis 5 Gewichts-% an Silber enthalten.

5. Verfahren nach Anspruch 4, wobei die Alginatfasern 0,2 bis 3,0 Gewichts-% an Silber enthalten.

6. Verfahren nach einem der Ansprüche 1 bis 5, wobei die Alginatfasern, zusätzlich zu dem Silbersalz, ein Salz oder Salze einschließen.

7. Verfahren nach Anspruch 6, wobei das zusätzliche Salz ein Zink-, Kupfer- oder Selensalz ist.

8. Verfahren nach einem der Ansprüche 1 bis 7, wobei die Fasern Alginat und wenigstens ein anderes Polysaccharid umfassen.

9. Verfahren nach Anspruch 8, wobei die Alginatfasern 70 bis 99 Gewichts-% an Alginat und 1 bis 30 Gewichts-% von dem anderen Polysaccharid umfassen.

10. Verfahren nach Anspruch 9, wobei das andere Polysaccharid Carboxymethylcellulose ist.

11. Verfahren nach einem der Ansprüche 1 bis 10, wobei die Fasern einen Durchmesser von 15 bis 20 Mikrometer haben.

12. Verfahren nach Anspruch 11, wobei das Silbersalz eine Teilchengröße von weniger als 5 Mikrometer hat.

13. Verfahren nach Anspruch 12, wobei das Silbersalz eine Teilchengröße von weniger als 2 Mikrometer hat.

14. Verfahren nach Anspruch 1, wobei die Spinnlösung, zusätzlich zu dem Alginat, wenigstens ein weiteres Polysaccharid einschließt.

15. Verfahren nach Anspruch 14, das die folgenden Schritte umfasst:
(a) das Bereiten einer wässrigen Spinnflüssigkeit, die weniger als 10 Gewichts-% von einem Feststoffgemisch enthält, das 70 bis 99 Gewichts-% an Alginat, 1 bis 30 Gewichts-% von dem wenigstens einen weiteren Polysaccharid und dem Silbersalz umfasst, und
(b) das Spinnen der Spinnlösung in ein Erstarrungsbad, das ein mehrwertiges Kation (vorzugsweise Kalzium) einschließt, um eine Vernetzung der Alginatketten zu bewirken.

16. Verfahren nach Anspruch 14 oder 15, wobei das weitere Polysaccharid Carboxymethylcellulose ist.

17. Verfahren nach Anspruch 1, wobei die Spinnlösung bereitet wird durch das anfängliche Dispergieren des Silbersalzes und jeglichen anderen Salzes in Wasser und danach das Lösen des Alginats und jeglichen anderen Polysaccharids.

18. Verfahren nach Anspruch 1, wobei die Spinnlösung bereitet wird durch das anfängliche Dispergieren des Silbersalzes und eines Zinksalzes und/oder Selensalzes und/oder Kupfersalzes in Wasser und danach das Lösen des Alginats und jeglichen anderen Polysaccharids.

## Revendications

1. Procédé de production de fibres d'alginate, comprenant les étapes ci-dessous:
(i) préparation d'une solution à filer contenant un polymère d'alginate dissous et une dispersion d'un sel d'argent particulaire, inorganique, insoluble dans l'eau, contenant au moins 50% en poids d'argent ; et
(ii) filature de ladite solution à filer dans un bain de coagulation contenant des cations polyvalents pour effectuer une réticulation des chaînes d'alginate.

2. Procédé selon la revendication 1, dans lequel le sel d'argent a une teneur en argent d'au moins 76% en poids d'argent.

3. Procédé selon la revendication 2, dans lequel le sel d'argent est un carbonate d'argent.

4. Procédé selon l'une quelconque des revendications 1 à 3, dans lequel les fibres d'alginate contiennent 0,1 à 5% en poids d'argent.

5. Procédé selon l'une quelconque des revendications 1 à 3, dans lequel les fibres d'alginate contiennent 0,2 à 3,0 % en poids d'argent.

6. Procédé selon l'une quelconque des revendications 1 à 5, dans lequel les fibres d'alginate incorporent un sel ou des sels, en plus dudit sel d'argent.

7. Procédé selon la revendication 6, dans lequel ledit sel additionnel est un sel de zinc, de cuivre ou de sélénium.

8. Procédé selon l'une quelconque des revendications 1 à 7, dans lequel les fibres comprennent de l'alginate et au moins un autre polysaccharide.

9. Procédé selon la revendication 8, dans lequel les fibres d'alginate comprennent 70 à 99% en poids d'alginate et 1 à 30% en poids dudit autre polysaccharide.

10. Procédé selon la revendication 9, dans lequel ledit autre polysaccharide est une carboxyméthylcellulose

11. Procédé selon l'une quelconque des revendications 1 à 10, dans lequel lesdites fibres ont un diamètre compris entre 15 et 20 microns.

12. Procédé selon la revendication 11, dans lequel le sel d'argent a une dimension des particules inférieure à 5 microns.

13. Procédé selon la revendication 12, dans lequel ledit sel d'argent a une dimension des particules inférieure à 2 microns.

14. Procédé selon la revendication 1, dans lequel la solution à filer incorpore au moins un polysaccharide additionnel, en plus de l'alginate.

15. Procédé selon la revendication 14, comprenant les étapes ci-dessous :
(a) préparation d'une solution à filer contenant moins de 10% en poids d'un mélange de solides, comprenant 70 à 99% en poids d'alginate, 1 à 30% en poids du au moins un polysaccharide additionnel et du sel d'argent ; et
(b) filature de la solution à filer dans un bain de coagulation incorporant un cation polyvalent (de préférence du calcium) pour effectuer une réticulation des chaînes d'alginate.

16. Procédé selon les revendications 14 ou 15, dans lequel ledit polysaccharide additionnel est une carboxyméthylcellulose.

17. Procédé selon la revendication 1, dans lequel la solution à filer est préparée en dispersant initialement le sel d'argent et un quelconque autre sel dans l'eau avant de dissoudre l'alginate et un quelconque autre polysaccharide.

18. Procédé selon la revendication 1, dans lequel la solution à filer est préparée en dispersant initialement le sel d'argent et un sel de zinc et/ou un sel de sélénium et/ou un sel de cuivre dans l'eau avant de dissoudre l'alginate et un quelconque autre polysaccharide.
